# EUROPEAN PATENT APPLICATION

(11) **EP 3 650 549 A1**
(43) Date of publication of application: **13.05.2020**
(21) Application number: 17916703.6
(22) Date of filing: 03.07.2017
(51) Int. Cl.: C12P 7/06, A23K 10/38

(54) **METHOD FOR PRODUCING ETHANOL AND FEED PRODUCTS**

(71) Applicant: JP Biotechnology, SIA, 3292 Jaunpagasts (LV)
(72) Inventor: BOSENKO, Anatolij, LV-2163 Carnikava (LV); SARGAUTIS, Darius, LV-2163 Carnikava (LV); SARGAUTIENE, Vanda, LV-2163 Carnikava (LV)
(74) Representative: Kromanis, Artis
(86) International application number: PCT/IB2017/054015
(87) International publication number: WO 2019/008413

(57) **Abstract**

The invention relates to the alcohol industry and the production of protein feed and may find application in the production of ethanol from cereal grains with the simultaneous production of feed products. The method for producing ethanol and feed products comprising the processing operations of grain grinding, recovering of brans and flour-grist mixture from the ground grain, enzymatic hydrolysis of flour-grist mixture to obtain mash, mash fermentation to obtain beer, the biomass containing mainly grain protein recovery from beer, the homogenization of biomasses, the removal of water soluble dry solids from the biomass.

## Description

The invention relates to the alcohol industry and the production of protein feed and may find application in the production of ethanol from cereal crops with simultaneous production of feed products.

There are known various methods for obtaining ethanol from cereal crops and recycling of waste products in feedstuffs. Method of processing liquid waste of alcohol production according to the patent of the Russian Federation Nº 2220195 can be outlined as an illustrative example. This method consists in the removal of suspended, colloidal and dissolved organic and mineral impurities from the stillage by means of physico-chemical methods, their precipitation, coagulation adding perlite, a water-soluble reagent polyelectrolyte and lime, after which the resulting precipitate is thickened and used as a feed product. Feed products obtained by such a method contain a large amount of fiber, added reagents and relatively small content (no more than 30% of the product weight) of crude protein, which determine their relatively low quality.

Various methods are also known for processing waste from alcohol production from cereal crops to produce feed products of higher quality for animals. For example, International application WO 2004/038031 describes a method of alcohol and protein feed production from cereals. According to this method, cleaned and ground grains are mixed with water, enzymatic hydrolysis is performed to produce mash, the sugar contained in the mash is fermented, and after beer distillation, a distillate and stillage containing suspended and dissolved solids are obtained.

The drawbacks of this method include low content of crude protein in feed product and low quality of protein, as well as low quality of feed product.

There are also known methods of producing ethanol and feed products from cereal crops to produce feed products with higher quality. A method to produce ethanol and feed products is described, for example, in patent Nº 13634 of the Republic of Latvia. According to this method techniques including grain grinding, recovery of brans and flour-grist mixture from the ground grain, enzymatic hydrolysis of flour-grist mixture to obtain mash, mash fermentation to obtain beer, beer separation to obtain clarified beer, drying biomass by heated inert gas to obtain feed products, as well as distillation steps of clarified beer and obtaining ethanol and stillage, processing stillage are set.

The drawbacks of this method include:
- the quality of feed products significantly reduces due to active interaction of unfermented reducing carbohydrates with amino acids in the drying process of biomass (the Maillard reaction), whose source is water-soluble dry solids of beer;
- a high content of water-soluble fiber in feed products negatively affects the content of crude protein;
- nucleic acids negatively influence the quality of feed products in case of drying biomass in combination with yeast grown using stillage;
- yeast growing using stillage and their extraction complicate technological process of production of ethanol and feed products.

Closest to the claimed method, adopted as a prototype, is a method of the ethanol and feed products production, implemented according to the patent No. 14966 of the Republic of Latvia including operations of grain grinding, the recovery of brans and flour-grist mixture from the ground grain, enzymatic hydrolysis of flour-grist mixture to obtain mash, mash fermentation to obtain beer, biomass containing mainly grain protein and grain fiber recovery from the beer, biomass containing mainly grain protein, biomass containing mainly yeast and biomass containing mainly fiber, drying of biomasses with a heated inert gas, as well as distillation, evaporation and stillage processing operations.

The main drawbacks of this method include:
- high content of water-soluble dry solids in feed products, much of which relates to fiber, negatively affect crude protein content and its quality in feed products;
- the quality of protein in feed products significantly reduces due to interaction of unfermented reducing carbohydrates with amino acids in the drying process of biomass (the Maillard reaction), whose source is water-soluble dry solids of beer;
- a high content of water-soluble dry solids in feed products limits it application in feed production;
- fiber recovery from beer prior to biomass recovery, which contains mainly grain protein, and the necessity of the subsequent utilization of fiber, complicates the technological process to obtain ethanol and feed products and reduces yield of feed products from processed grains.

The aim of the proposed solution is to develop a method to obtain the following feed products with distinctive positive properties:
- a feed product containing mainly of grain protein; with a lower content of water-soluble dry solids, with a higher content of crude protein and its quality,
- a feed product containing mainly yeasts with a low content of water-soluble dry solids, with a higher content of crude protein and its quality ;
- a feed product containing mainly grain protein and yeasts with a low content of water-soluble dry solids; with a higher content of crude protein and its quality;
- a feed product containing biologically active components of enzymatically degraded yeasts (unbound beta-glucan, mannan , nucleotides, amino acids, peptides and polypeptides) and accompanying degraded yeast components of the enzymatic hydrolysis of grain protein (amino acids, peptides, polypeptides), hereinafter referred to as the degraded yeasts, with a low content of water-soluble dry solids, with a high content of crude protein and its quality;
- a feed product containing mainly grain protein and biologically active components of degraded yeasts, with a low content of water-soluble dry solids, with a high content of crude protein and its quality .

The objective to obtain the feed product with distinctive positive properties, containing mainly grain protein with a low content of water-soluble dry solids, with a higher content of crude protein and its quality; to obtain the feed product with distinctive positive properties containing mainly yeasts with a low content of water-soluble dry solids, with a higher content of crude protein and its quality; to obtain the feed product with distinctive positive properties containing mainly grain protein and yeasts with a low content water-soluble dry solids, with a higher content of crude protein and its quality; to obtain the feed product with distinctive positive properties containing biologically active components of enzymatically degraded yeasts with a low content of water-soluble dry solids, with a high content of crude protein and its quality; to obtain feed product with distinctive positive properties containing mainly grain protein and biologically active components of degraded yeast, with a low content water-soluble dry solids, with a high content of crude protein and its quality is achieved by that the method for producing ethanol and feed products comprising the processing operations of grain grinding, recovering of brans and flour-grist mixture from the ground grain, enzymatic hydrolysis of flour-grist mixture (FGM) to obtain mash, mash fermentation to obtain beer, the recovery from beer the biomass containing mainly grain protein, and the biomass containing mainly yeasts, drying of biomasses with a heated inert gas at a temperature of 110-160°C, as well as distillation of beer and beer distillate, evaporation and stillage processing, from the biomass containing mainly grain protein and is recovered from beer the dry feed product is obtained with a low content of water-soluble dry solids, with a higher content of crude protein and its quality, by removing water-soluble dry solids from biomass in the amount of 65-85% of their content in biomass, in addition from the biomass containing mainly yeasts and is recovered from beer the dry product is obtained with a low content of water-soluble dry solids, with a higher content of crude protein and its quality, by removing water-soluble dry solids from biomass in the amount of 75-95% of their content in biomass, in addition from the biomass containing mainly grain protein and is recovered from beer and from biomass containing mainly yeasts and is recovered from beer the dry product is obtained containing mainly grain protein and yeast, with a low content of water-soluble dry solids, with a high content of crude protein and its quality by removing water-soluble dry solids from biomass containing mainly grain protein in the amount of 65-85% of their content in biomass and by removing water-soluble dry solids from biomass containing mainly yeasts in the amount of 75-95% of their content in biomass, in addition from the biomass containing mainly yeasts and is recovered from beer a dry product is obtained containing biologically active components of degraded yeasts with a high content of crude protein and its quality by removing water-soluble dry solids from the biomass containing mainly yeast in the amount of 75-95% of their content in biomass, with subsequent enzymatic degradation of yeasts to biologically active components, in addition from the biomass containing mainly grain protein and is recovered from beer, and from biomass containing mainly yeasts and is recovered from beer, a dry product is obtained with a low content of water-soluble dry solids, with a high content of crude protein and its quality, with biologically active components of degraded yeasts, by removing water-soluble dry solids from biomass containing mainly yeasts in the amount of 75-95% of their content in biomass, with subsequent enzymatic degradation of yeasts to biologically active components and by removing water-soluble dry solids from biomass containing mainly grain protein in the amount of 65-85% of their content in biomass.

The source of the water-soluble dry solids (WSDS) in the biomass containing mainly grain protein and is recovered from beer, and in biomass containing mainly yeast is beer, the content of which in recovered biomasses is in range of 76-86%. WSDS in beer comprise mainly: non fermented reducing carbohydrates and dextrins, formed during the enzymatic hydrolysis of non-starch polysaccharides - 1.2-1.7%; minerals -0.3-0.6%; glycerin - 0.4-0.7 %; other substances - 0.2-0.5 %. Drying of recovered biomasses from beer, without prior removal of WSDS, causes the following negative indicators of feed products derived from biomasses: a lower protein content in feed products due to high content of water-soluble dry solids; reduced quality of crude protein in feed product due to interaction of non-fermented reducing carbohydrates with amino acids in the drying process of biomass (the Maillard reaction); limited scope of application, as a result of mentioned above negative indicators.

In the proposed solution, in obtaining all the above listed feed products with distinctive positive properties, it is determined to remove a significant part of the WSDS from biomasses recovered from the beer before their drying. The following is a description of the processes associated with obtaining feed products of the above with distinctive positive properties.

### Production of the beer

Cleaned grain, preferably wheat, triticale or a mixture thereof, containing over 11 % of crude protein, on a dry matter (DM) basis, is ground by known techniques and then separated by known techniques into two fractions - FGM and brans. The most preferred concentration of crude protein in the grain as a feedstock is 13.0-15.0% DM. Grain processing with such a concentration of crude protein in the grain allows to obtain above listed feed products with distinctive positive characteristics containing crude protein 66-70% by weight with moisture content of the products ranging 5-8%. The proposed solution also provides the method of grain processing and with less content of crude protein (11 -13% DM). Moreover, due to the removal of WSDS from biomass recovered from beer, the crude protein content in feed products with distinctive positive properties will also be at a relatively high level 60 -66% by weight of the product, with the moisture content of products in range of 5-8% . The proposed solution also provides the possibility to process cereal grains with crude protein level below 11% DM. Thus, by removing WSDS from the recovered biomasses from beer, the concentration of crude protein in feed products will be relatively higher then compared to the feed products obtained from biomasses without removing WSDS. However, processing grain containing crude protein lower than 11% DM will lead to lowering of crude protein in the dry products and their yield. The proposed solution provides the recovery of FGM, which contains the insoluble fiber in the range less than 0.8-1.2 % DM. Processing of FGM with a low fiber content allows to exclude the step of fiber recovery from beer (provided in the prototype), prior to the step of the recovery from beer the biomass containing mainly grain protein, in addition the step of utilization of the recovered fiber is excluded. The resulting FGM, comprising mainly starch, crude protein and small amounts of mineral substances, fiber, fat and other is mixed with the aqueous medium and enzymes - sources of alpha amylase, xylanase and beta - glucanase. The proposed embodiment at the step of enzymatic hydrolysis of FGM provides the use of water or mixture of water and aqueous medium as an aqueous medium, formed in processing ethanol and feed products. It is more preferable to use a mixture of water, bottoms and stillage obtained by distillation of aqueous-ethanol solution (see below - distillation of clarified beer and aqueous-ethanol solution containing WSDS). In the process of enzymatic hydrolysis of the FGM , hydrolyzate is formed. Then, nutrient salts and glucoamylase are added to the hydrolyzate. The resulting mash is fermented by using yeast by known methods resulting in alcoholic beer, containing ethanol 9-16% by volume of beer (preferably 11-13%), WSDS -2.3-3.4 % by weight of beer (preferably 2.5- 2.7%) and total suspended solids (TTS) ranging 4,8-5,8 % by weight of the beer (preferably 5.1-5.4 %). TTS of beer mainly comprise grain protein (63-70 %), yeasts (15-25 %), a small amount of fiber (7-9 %) and other substances.

From the resulting beer in subsequent steps, biomass containing mainly grain protein and biomass containing mainly yeasts are recovered, with their subsequent use in obtaining the above feed products with distinctive positive properties.

### Obtaining the feed product containing mainly grain protein.

In the proposed solution of obtaining the feed product containing mainly grain protein, there is provided to recover from beer the biomass containing mainly grain protein, followed by the removal of WSDS from biomass and biomass drying. The biomass is recovered by a known methods, for example, by separation using a decanter centrifuge. This produces the biomass containing 16 -26% DM, preferably - 19-22%, and the beer containing the main part of the yeasts, small amount of the accompanying grain protein and WSDS. DM of the recovered biomass contains 60-64 % crude protein, 7-9 % fiber, 8-15 % WSDS, 5-7% fat and a small amount of residual yeasts. The proposed solution to remove WSDS from the recovered biomass provides the utilization of water, different aqueous media formed in the ethanol and feed product production processes, which do not contain WSDS or concentration of WSDS in which are significantly lower than in recovered biomass. It is the most preferable for WSDS removal from biomass to use aqueous-ethanol solution, obtained in the step of WSDS removal from biomass containing mainly yeasts (see below - obtaining the feed product containing mainly yeasts). The biomass is mixed with aqueous-ethanol solution in the ratio 1.0:2.2-2.8, preferably -1.0:2.4-2.6), and from the resulting suspension biomass by known technics are recovered, for example, by separating using a decanter centrifuge. The obtained biomass containing 18-26% DM (preferably - 20-23%) is dried, while obtained aqueous-ethanol solution containing 0.7-1.2% WSDS is provided to distillation, in mix with clarified beer or separately. The proposed solution provides to dry the obtained biomass by known methods using known dryers, for example, a fluid bed dryer. As a heat carrier, at the inlet to the dryer, carbon dioxide heated to a temperature 110-160°C is used. At the exit from the dryer temperature of the mixture of CO2 and water ethanol vapor is maintained in the range of 75 - 100°C. After dryer the feed product is obtained in the form of granules, with a moisture content of 3-10 % (preferably 5-8%). Removal of WSDS from biomass in an amount of 65 -85 % of their content in biomass results in the reduced content of WSDS in the feed product, consequently increases the relative content of crude protein in it by 7-14% (optimally 9-12 %). Thus, the quality of the protein in the feed product increases significantly due to the removal of reducing carbohydrates from the biomass, which can react with amino acids of proteins during the drying process (the Maillard reaction). In the process of the biomass drying, water-ethanol condensate is recovered, part of which is taken from the dryer and used to remove WSDS from biomasses containing mainly yeast and grain protein. Another part of the condensate is used to regenerate CO2, which is used as a heat carrier in the dryer loop (hereinafter CO2 regeneration). The proposed solution also provides the conservation of biomass before its drying by known methods, for example, by adding ethanol to biomass in range 9-15% by volume (preferably -11-13%).

### Obtaining the feed product containing mainly yeasts.

In the proposed solution of obtaining the feed product containing mainly yeast, there is provided to recover from beer the biomass containing mainly yeast, from which the biomass containing mainly grain protein had already been recovered. Then from the recovered biomass it is provided to remove WSDS with subsequent drying of biomass. The biomass containing mainly yeast is recovered from beer by known techniques, for example, by separation using a sedicanter. The recovered biomass contains 13 -20% DM (preferably 14-16%). DM comprises 45 -60 % yeast (preferably -50-55%), 15-25% grain protein and 12-20% WSDS (preferably -14-16 %). The proposed solution to remove WSDS from biomass containing mainly yeasts, in the amount of 75-95% of the their content in biomass (preferably - 80-90%), provides the usage of water-ethanol condensate recovered in the drying process, separately or in mixture with liquids, recovered from distillation and feed product production steps. Aqueous liquids should not contain WSDS or their content should be significantly lower than WSDS content in biomass. It is the most preferable to use the aqueous-ethanol solution comprising water-ethanol condensate recovered from the drying of biomasses step and water condensate recovered from the stillage evaporation step in the ratio 1.0:2.7-3.4 (preferably 1.0:2.9-3.1). The biomass is mixed with aqueous-ethanol solution in the ratio 1.0:8.0-12.0 (preferably 1.0:9.5:10.5) then from the obtained suspension the biomass is recovered by conventional means, for example, by sedicanter. Recovered biomass, containing 13-20% DM (preferably 15-17%) is passed to drying, yet aqueous-ethanol solution containing 0.2-0.4% WSDS is used in removal of WSDS from the biomass containing mainly grain protein (ref. above in obtaining the feed product containing mainly grain protein). The recovered biomass is provided to drying step by conventional means, for example, by fluid bed dryer. As a heat carrier, at the inlet to the dryer, carbon dioxide heated to a temperature 110-160°C is used. At the exit from the dryer the temperature of the mixture of CO2 and water-ethanol vapor is maintained in the range of 75 - 100°C. After dryer the feed product is obtained in the form of granules, with a moisture content of 3-10 % (preferably 5-8%). Removal of WSDS from biomass in an amount of 75 -95 % of their content in biomass results in the reduced content of WSDS in the feed product, consequently increases the relative content of crude of protein in it by 12-18% (preferably 14-16%). Thus, the quality of the protein in the feed product increases significantly due to the removal of reducing carbohydrates from the biomass that can react with amino acids of proteins during the drying process (the Maillard reaction). In the process of the biomass drying, water ethanol condensate is recovered, some amount of which is collected and used to remove WSDS from biomasses containing mainly yeast and grain protein. Another part of the condensate is used to regenerate CO2. The proposed solution also provides the conservation of biomass before its drying by known methods, for example, by adding ethanol to biomass in range 9-15% by volume (preferably -11-13%). The proposed solution also provides an option of yeast thermolysis in biomass before its drying. Thermolysis is performed at a temperature in the range 60-80°C (optimally 65-70°C) whilst the biomass is hold in a buffer tank.

### Obtaining the feed product containing mainly grain protein and yeast.

In the proposed solution of obtaining the feed product containing mainly grain protein and yeasts there is provided to recover from beer the biomass containing mainly grain protein and to recover from beer the biomass containing mainly yeast, with subsequent removal of WSDS from biomasses, mixing of the recovered biomasses and their drying. The recovering from beer the biomass containing mainly grain protein followed by the removal of WSDS from biomass in the range of 65-85% of their content in biomass is performed identically as mentioned above (see above - obtaining the feed product containing mainly grain protein). The recovering from beer the biomass containing mainly yeast followed by the removal of WSDS from biomass in the range of 75-95% of their content in biomass is performed as mentioned above (see above - obtaining the feed product containing mainly yeast). The resulting biomasses are fed to mixer, then the resultant mixture is fed to a buffer tank. The resulting mixture of biomasses comprising 14-20% DM ( preferably 16-18%) is dried by conventional techniques in known dryers, for example, in fluid bed dryer. As a heat carrier, at the inlet to the dryer, carbon dioxide heated to a temperature 110-160°C is used. At the exit from the dryer the temperature of the mixture of CO2 and water-ethanol vapor is maintained in the range of 75 - 100°C. After dryer the feed product is obtained in the form of granules, with a moisture content of 3-10 % (preferably 5-8%). Removal of WSDS from biomasses in an amount of 70 -85 % of their content in biomass (preferably 72-77%) results in the reduced content of WSDS in the feed product, consequently increases the relative content of crude of protein in it by 10-14% (preferably 8-12%). Thus, the quality of the protein in the feed product increases significantly due to the removal of reducing carbohydrates from the biomass that can react with amino acids of proteins during the drying process (the Maillard reaction). In the process of the biomass drying, water-ethanol condensate is recovered, some amount of which is collected and used to remove WSDS from biomasses containing mainly yeast and grain protein. Another part of the condensate is used to regenerate CO2. The proposed solution also provides the conservation of biomass before its drying by known methods, for example, by adding ethanol to biomass in the range of 9-15% by volume (preferably -11-13%).

### Obtaining the feed product containing components of the degraded yeast.

In the proposed solution of obtaining the feed product containing the components of the degraded yeast there is provided to recover from beer the biomass containing mainly yeast, followed by the removal of WSDS, degradation of the yeast to biologically active components (free beta-glucan and mannan, nucleotides) and hydrolysis most of intracellular yeast proteins and grain protein, accompanying yeast, to amino acids, peptides and polypeptides, with subsequent drying of the biomass. The recovery of yeast biomass from beer followed by the removal of WSDS is performed identically as mentioned above (see above - obtaining a feed product predominantly comprising yeasts). The resulting yeast biomass containing 13-20% DM (preferably 14-16%) is diluted with an aqueous medium, for example with bottoms, to its content of 7-12% DM (preferably - 8-10%) and initially nucleic acids reduction is performed during 40-90 min (preferably 60-70min) at pH 6.5-8.0 (preferably 6.9-7.5) and temperature 50 - 60°C (preferably 52 - 56°C). Thus, 70-90% of yeast nucleic acids are hydrolysed mainly to nucleotides. Then, alkaline protease is added into suspension and yeast degradation has been continued for 2-10 hours (preferably to 4-6 hours) at pH in the range of 7.0-10.0 (preferable 7.5-8.5). Thus, yeast cells in the range of 80-90% are degraded to unbound beta-glucan and mannan. Main part of intracellular yeast protein, in addition to grain protein which accompanies yeasts, is hydrolyzed mainly to amino acids, peptides and polypeptides. DM of the obtained suspension comprises crude protein in the range of 67-70%, beta-glucan 5-6%, mannan 3.5-4.5%, nucleotides 2.0-3.0%, as well as fat and minerals.

The suspension of the degraded yeast is dried by conventional techniques in known dryers, for example, in spray dryer. When dry the suspension, a heat carrier at the inlet to the dryer, carbon dioxide heated to a temperature 150-250°C is used. At the exit from the dryer the temperature of the mixture of CO2 and water-ethanol vapor is maintained in the range of 75 - 100°C. After dryer the feed product is obtained in the form of dust-like particles, with a moisture content of 7-10 %. The resulted feed product has the following positive characteristics: low content of WSDS and high content of crude protein ranging in the level of 60-72 % (preferably 64-70%); polypeptides and peptides are of high quality due to removing reducing carbohydrates in the range of 75-95% of their content in biomass (preferably 80-85%), which can react with amino acids of these compounds (Maillard reaction); peptides, polypeptides, unbound beta-glucan (4.5-5.5%) and mannan (3-4%), as well as nucleotides (2-3%) positively affect the quality of the feed product. During the drying of the suspension the water- ethanol condensate forms. A part of the formed condensate with the ethanol concentration of about 1% by volume is taken from a drying unit and is used for various purposes in the production of feed products and ethanol, for example, when removing WSDS from biomasses. Another part of the condensate is used for CO2 regeneration. In the proposed solution is provided to perform yeast degradation in yeast biomass without its prior dilution with aqueous media, as well as applying neutral and acid proteases to degrade yeast cell walls, to hydrolyse grain and yeast protein. However, in this case, the implementation of the technological process will become more complicated and the processes associated with the degradation of yeast will slow down.

### Obtaining the feed product containing mainly grain protein and components of degraded yeast.

In the proposed solution of obtaining the feed product containing mainly grain protein and components of degraded yeasts there is provided to recover from beer the biomass containing mainly grain protein and the biomass containing mainly yeast, followed by the removal of WSDS from the recovered biomasses, yeast degradation in the yeast biomass, mixing biomasses and drying the resulting mixture. The recovering from beer the biomass containing mainly grain protein followed by the removal of WSDS from biomass in the range of 65-85% of their content in biomass is performed identically as mentioned above (see above - obtaining the feed product containing mainly grain protein). The recovering from beer the biomass containing mainly yeast followed by the removal of WSDS from biomass in the range of 75-95% of their content in biomass is performed identically as mentioned above (see above - obtaining the feed product containing mainly yeast). Yeast degradation in biomass containing mainly yeast is performed identically as mentioned above (see above - obtaining the feed product containing components of the degraded yeast). The biomass containing mainly grain protein and suspension of degraded yeast are mixed by mixer and then passed to a buffer vessel. The obtained mixture of biomasses containing 12-18% DM (preferably 14-16%) is dried by conventional techniques in known dryers, for example, in fluid bed dryers. As a heat carrier, at the inlet to the dryer, carbon dioxide heated to a temperature 110-160°C is used. At the exit from the dryer the temperature of the mixture of CO2 and water-ethanol vapor is maintained in the range of 75 - 100°C. After dryer the feed product is obtained in the form of granules, with a moisture content of 3-10 % (preferably 5-8%). The obtained feed product demonstrates following positive properties: low content of WSDS and high crude protein content in the range of 60-68% of product weight (preferably 63-67%); polypeptides and peptides are of high quality due to removing reducing carbohydrates in the range of 65-80 % of their content in biomass (preferably 70-75%), which can react with amino acids of these compounds during drying biomass (Maillard reaction); amino acids, peptides, polypeptides, unbound beta-glucan (1.0-1.3 % of product weight) and mannan (0.6-0.8% of product weight), as well as nucleotides (0.4-0.6% of product weight) positively affect the quality of the feed product. During the drying of the mixture of biomasses the water- ethanol condensate forms, part of which is taken from a drying unit and is used in removing WSDS from biomasses containing mainly yeast and grain protein. Another part of the condensate is used for CO2 regeneration. In the proposed solution is provided the conservation of the mixture of the biomasses before drying by conventional means, for example, by adding ethanol into biomass to its content in the mixture in the range of 9-15% by volume (preferably 11-13%).

### Distillation of clarified beer and aqueous-ethanol solution containing WSDS.

The proposed solution provides to distill clarified beer and aqueous-ethanol solution containing WSDS in mixture or separately by known techniques and equipment. It is most preferable to distill beer and aqueous-ethanol solution separately. This makes it possible to obtain stillage with a higher DM content and reduce the energy consumption for its evaporation. By distillation of a clarified beer containing 9-16% of ethanol by volume (preferably 11-13%) the stillage and beer distillate are obtained. The stillage passes to evaporation, and the beer distillate is passed to rectification. By rectification of the beer distillate mixed with ethanol condensate by known techniques and equipment, the ethanol and bottoms are obtained. Bottoms is utilized by conventional means, for example, using in the enzymatic hydrolysis of FGM in the mixture with water. By distillation of the aqueous-ethanol solution, containing WSDS, the ethanol condensate and water solution, containing WSDS, are obtained. The proposed solution provides for the resulting water solution, containing WSDS, to be used in a mixture with water or other aqueous media during the enzymatic hydrolysis of FGM, and ethanol condensate to be rectified in a mixture with the beer distillate.

### The utilization of the stillage and brans.

The proposed solution provided the utilization of the stillage and brans by known techniques and equipment. For example, the stillage containing 2.3-3.4% DM (preferably 2.6-3.0%) mainly comprising WSDS is concentrated by known techniques and conventional evaporating equipment to 45-85% DM (preferably 55-65%), then mixed with brans in a ratio of 1.0: 1.5-1.7. The resulting mixture containing 75-80% DM is dried by known techniques and conventional drying equipment, for example, by drum dryer. In the drying process the feed product is obtained in the form of dispersive particles with a moisture content of 7-12%, with crude protein concentration in a range of 15-18% by weight of the product. The proposed solution also provides an option for obtaining the feed product in the form of granules. The water condensate, obtained in the processes of the stillage evaporation, and in the drying of the mixture of concentrated stillage and brans, is used in the enzymatic hydrolysis of FGM or is utilized by other ways, for example, is sewage treatment plants. The proposed solution also provides other options of stillage utilization. For example, the stillage is concentrated to 50-85% DM and used as fuel in a steam boiler, for example, in a mixture with wood residuals. It is provided an option to concentrate the stillage to 10-45% DM, with its subsequent use in biogas production. The proposed solution also provides various known methods for utilizing bran, for example, their granulation with subsequent use in feed production.

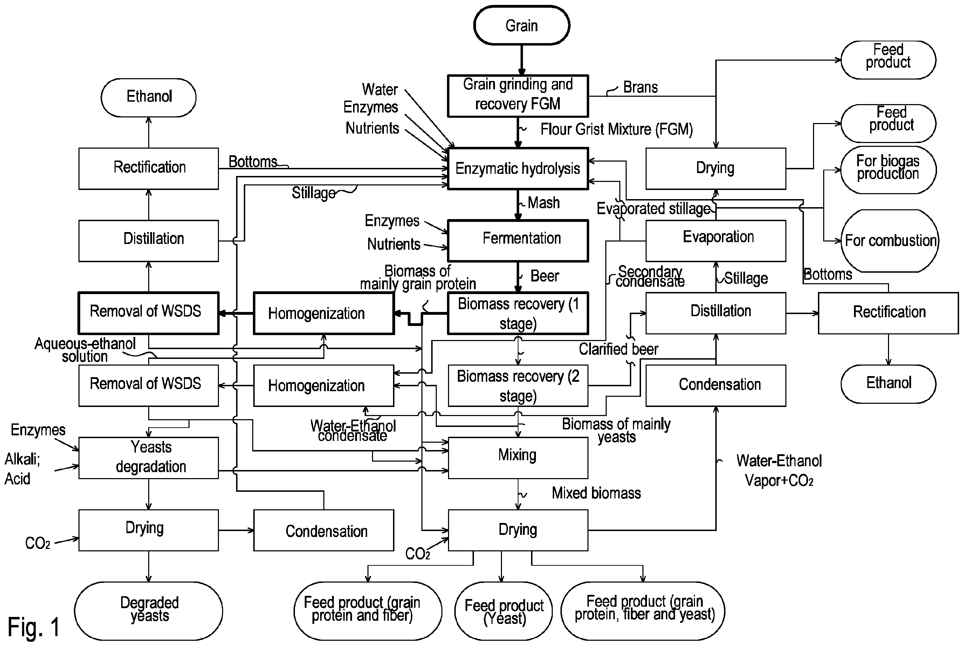

Figures 1-9 (Fig. 1 - 9) illustrate the description above.

Fig. 1 is a block diagram, illustrating a method for production ethanol and a feed products comprising grain grinding operation, recovering from the grinded grain flour-grist mixture and bran, enzymatic hydrolysis of the flour-grist mixture to obtain the mash, the fermentation of the mash and obtaining the beer, recovering from the beer the biomass, containing mainly grain protein, characterized in that from the biomass, containing mainly grain protein and is recovered from beer, WSDS are removed in an amount of 65-85%, preferably 70-80%, of the total WSDS contained in the biomass recovered from the beer.

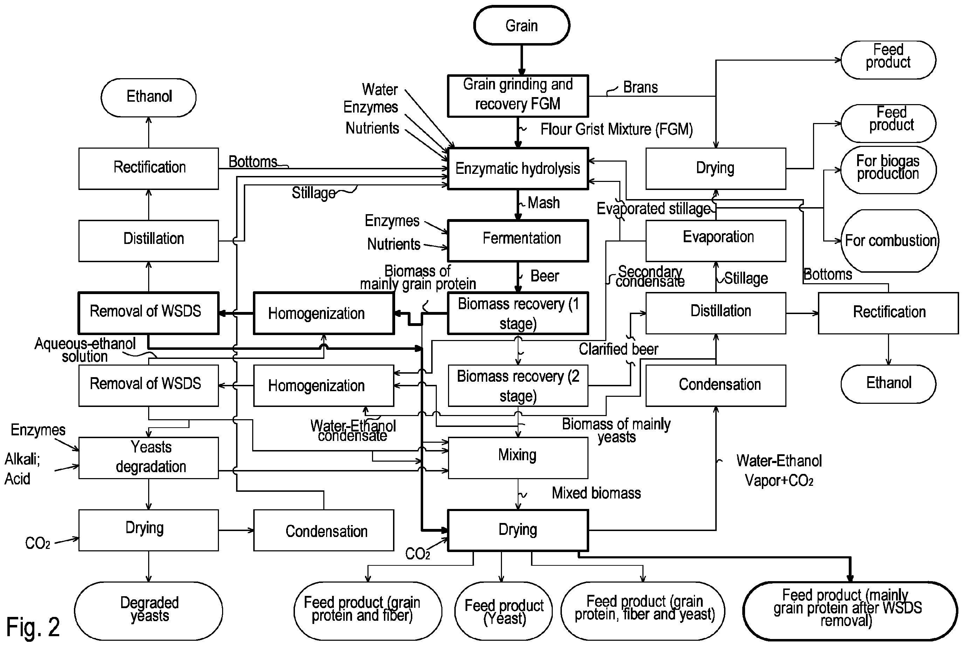

Fig. 2 is a block diagram, illustrating a method, where the biomass recovered from beer, containing mainly grain protein, after the removal of WSDS is dried obtaining the feed product with a moisture content in the range of 3-10% , preferably 5-8%, comprising crude protein 58-72% by weight in the product, preferably 60-68%.

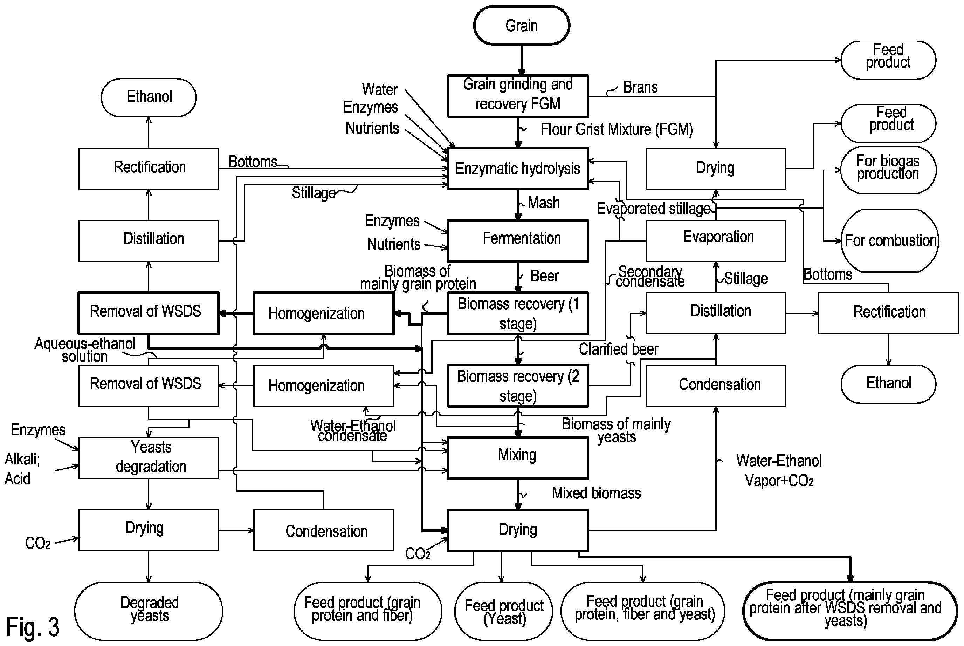

Fig. 3 is a block diagram, illustrating a method where the additional recovery from beer the biomass containing mainly yeasts is performed, followed by mixing this biomass with the biomass containing mainly grain protein, in which WSDS have been removed in the amount of 65-85% of their content in the biomass (preferably 70-80%) and fed the mixture of these biomasses to the dryer, obtaining a feed product with a moisture content in the range of 3-10%, preferably 5-8%, comprising crude protein 58-72% by weight in the product, preferably 60-68%.

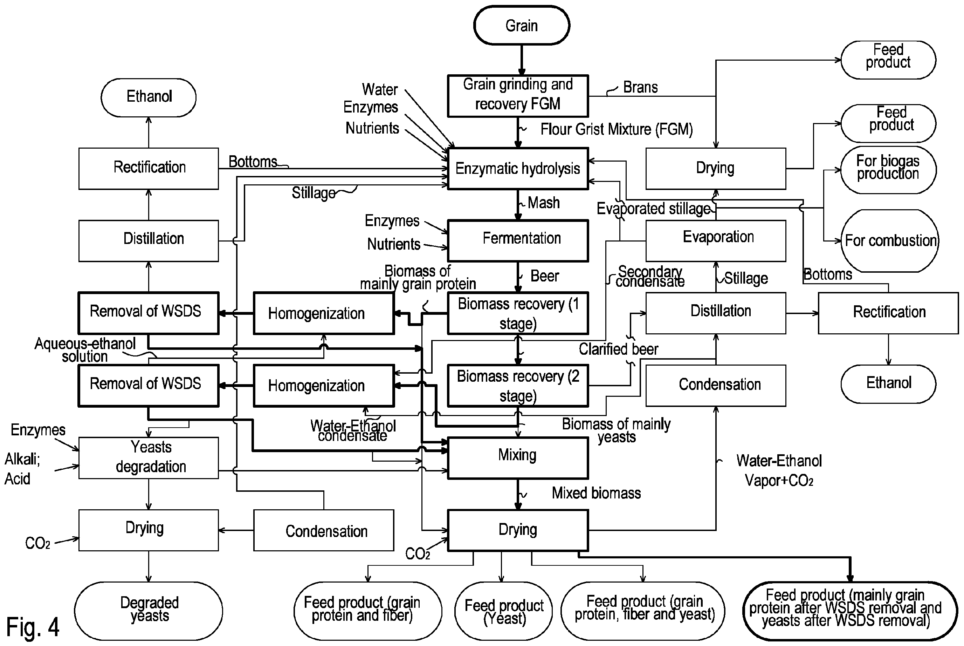

Fig. 4 is a block diagram illustrating a method where WSDS in an amount of 75-95% of their content in the biomass, preferably 80-90%, are removed from the biomass, containing mainly yeast and is recovered from the beer, followed by mixing this biomass with the biomass containing mainly grain protein in which the amount of WSDS have been removed in an amount of 65-85% of their content in the biomass, preferably 70-80%, and passing the mixture of these biomasses into drying, obtaining a feed product containing of 58-72%, preferably 60-68% by weight of crude protein in the product.

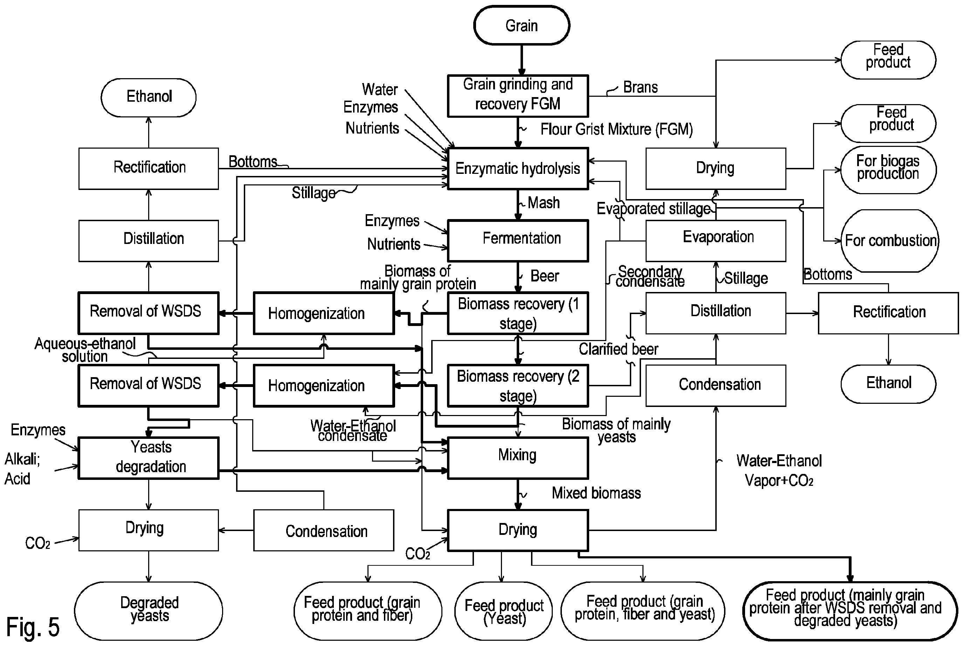

Fig. 5 is a block diagram , illustrating a method where in the biomass containing mainly yeasts and is recovered from beer, after WSDS removal in an amount of 75-95% of their content in the biomass, preferably 80-90%, further degradation of the yeast is performed to the biological active components and hydrolysis of the grain protein, accompanying yeast, to amino acids, peptides and polypeptides, followed by mixing the resulting suspensions with biomass containing mainly grain protein in which the amount of WSDS are removed in an amount of 65-85% of their content in the biomass, preferably 70-80%, and the mixture of these biomasses is dried, obtaining a feed product with a moisture content of 3-10% (preferably 5-8%), comprising crude protein in the product in the range of 58-72% by weight, preferably 60-68%.

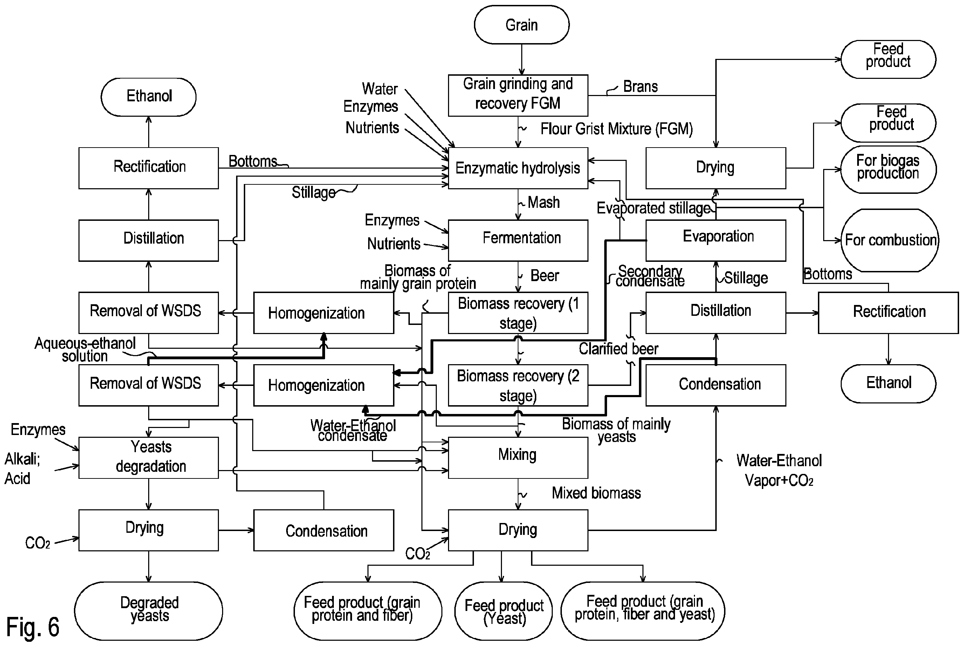

Fig. 6 is a block diagram illustrating a method where to remove WSDS from the biomass containing mainly yeast, an aqueous-ethanol solution obtained by mixing the water condensate obtained by evaporating the stillage and the water-ethanol condensate obtained by drying the biomasses, and to remove WSDS from the biomass containing mainly grain protein, an aqueous-ethanol solution, obtained by removing WSDS from the biomass containing mainly yeast is used.

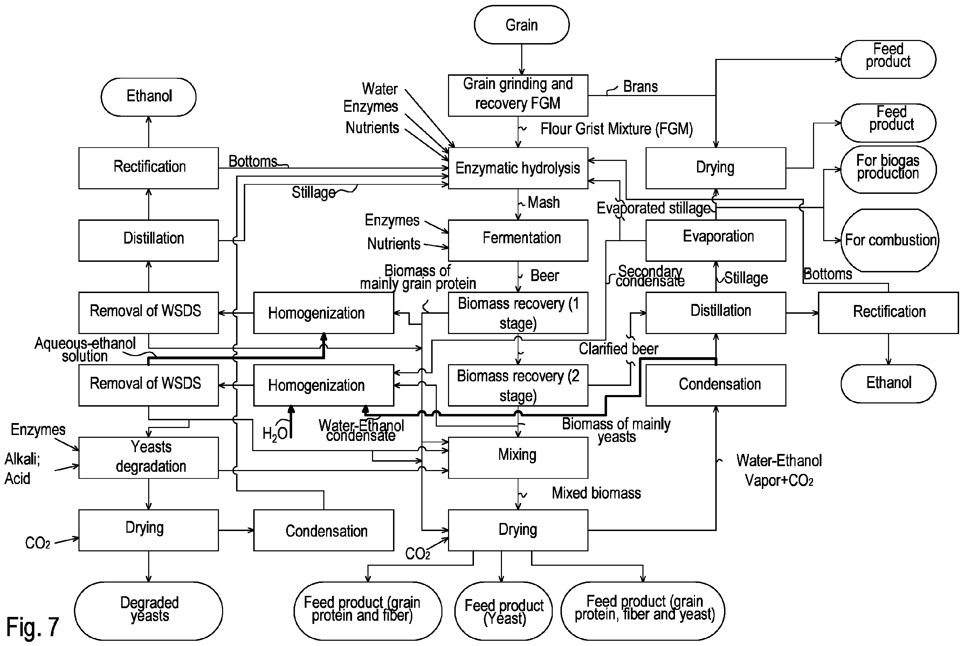

Fig. 7 block diagram illustrating a method where to remove WSDS from a biomass containing mainly yeasts, an aqueous-ethanol solution obtained by mixing water and water-ethanol condensate obtained by drying the biomasses is used, and to remove WSDS from a biomass containing mainly grain protein, an aqueous-ethanol solution obtained by removing WSDS from a biomass containing mainly yeast is used.

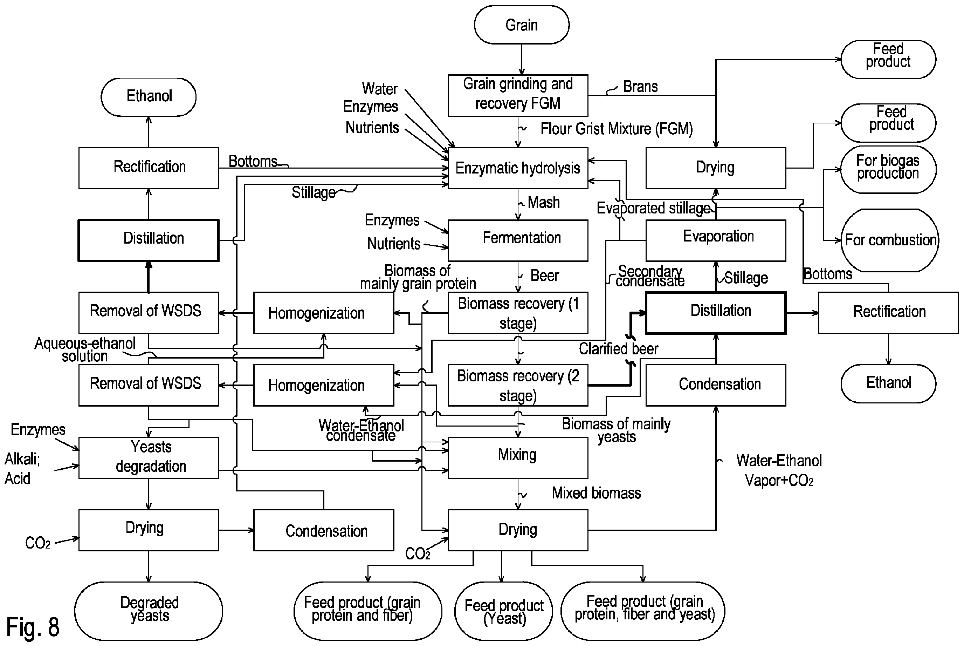

Fig. 8 block diagram illustrating a method where the aqueous-ethanol solution obtained by removing WSDS from the biomasses is distilled separately from the clarified beer, and the resulting aqueous solution containing mainly WSDS is used in the enzymatic hydrolysis of the flour-grist mixture.

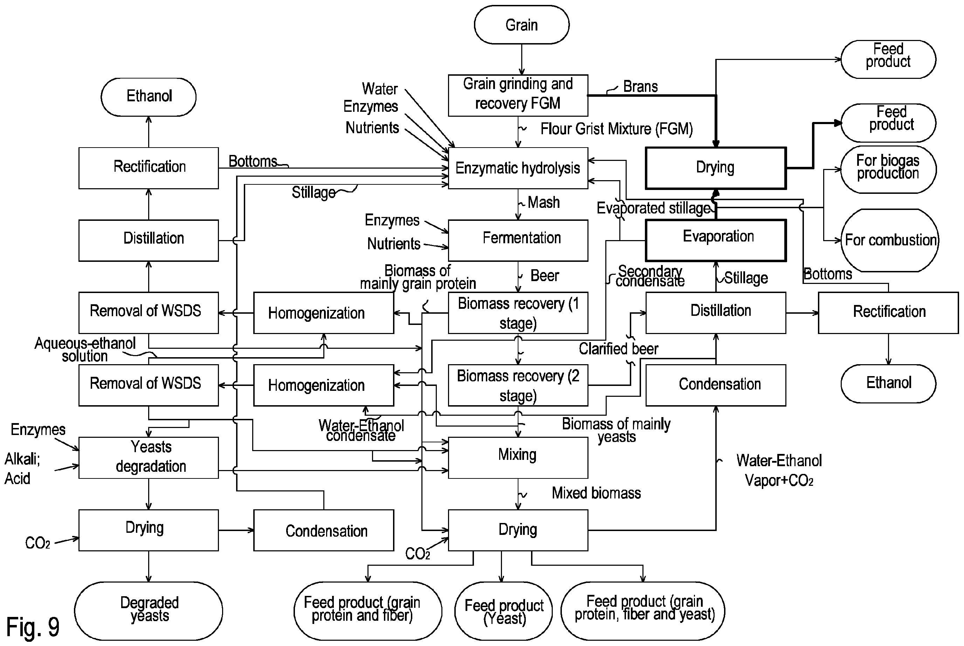

Fig. 9 block diagram illustrating a method where the stillage is evaporated to a DM content of 50-85% and then the evaporated stillage is dried together with bran to obtain a feed product.

Implementation example 1: by reducing WSDS, increasing the content of crude protein and its quality in the feed product containing mainly grain protein and in the feed product containing mainly yeast, by removing the WSDS from the biomasses recovered from the beer.

### Grinding grain, recovering the flour-grist mixture and bran from the grinded grain.

Starting feedstock of wheat with a moisture content of 12.7-12.9% with a crude protein content of 13.7-14.5 % DM is cleaned from accompanying impurities and moistened to 14.2-14.6% to develop the elasticity of the bran, which prevents their destruction into small parts during grinding and transition to a flour-grist mixture (hereinafter-FGM) during fractionation. The moistened grain is crushed using roller mills and the resulting grinded grain is recovered by known means to FGM with a particle size of up to 0.8 mm and brans. The resulting FGM with a moisture content of 13.2-13.7% is used to produce mash, while brans are used in the production of dry feed product containing mainly crude protein and carbohydrates.

### Enzymatic hydrolysis of FGM and mash production.

The FGM and the mixture of water and bottoms are fed continuously in a ratio of 1.0 : 2.6-2.8 to the first stage hydrolysis vessel. Simultaneously, into the hydrolysis vessel xylanase enzyme preparation (Sunson, CN, activity 6 000 000 units / ml) (source of xylanase and beta glucanase) in an amount of 0.008-0.014% and the enzyme preparation alpha amylase HTAA (Sunson, CN, activity 30 000 units / ml) (source of thermostable alpha amylase) in an amount of 0.01-0.030% of the suspension weight, are continuously fed. Hydrolysis of the FGM of the obtained suspension is performed at a temperature range of 57-62°C for 15-20 minutes at a pH range of 6.0-6.5. The resulting suspension is continuously fed to the second stage hydrolysis vessel, where it is heated with a direct steam to the temperature of 90-95°C and the enzymatic hydrolysis of FGM starch to dextrins is continued. The duration of hydrolysis at the specified temperature is 50-60 minutes, then to the obtained hydrolysate nutrient salts are added. The hydrolysate is cooled to a temperature of 28-32°C. Then, the enzyme preparation of glucoamylase (Sunson, CN, activity 150,000 units/ml) (source of glucoamylase) is added to the hydrolysate in an amount of 0.015-0.030% by weight of the hydrolysate. The resulting mash, with a dry matter concentration of 23.5-25.0%, is used in the preparation of yeast propagation and beer.

### Fermentation of the mash and obtaining of beer.

Fermentation of the mash by yeast to ethanol is performed by a batch process. In this method, the mash, cooled to a temperature of 28-32°C , is fed into a batch fermenter, where propagated yeast is simultaneously fed in an amount of 8.0-10% of the volume of the mash in the fermenter.

The fermentation process is maintained at a temperature of 28 - 32°C for 66 -72 hours. When the process of fermentation of the mash is completed, the beer is formed, which contains the ethanol in an amount of 11-13% by volume and small amounts of the other alcohols (methanol, propanol, etc.), as well as suspended (5,1-5,4%) and water-soluble dry solids (2.5 - 2.7%) - proteins, fiber, yeasts, reducing non-fermented carbohydrates, dextrins formed from non-starch polysaccharides, glycerin, minerals, fat and small amounts of peptides and polypeptides, volatile organic acids, acid salts. During fermentation, carbon dioxide is formed, which is used as a heat carrier for drying biomasses.

### The biomass containing mainly grain protein recovery from beer and the removal of the WSDS from the biomass.

The biomass, containing mainly grain protein, is recovered from the beer by separation using a decanter. Thus, the biomass containing 19-22% of dry matter and beer containing the major part of yeasts, a small part of grain protein and WSDS is obtained. The dry matter of the recovered biomass comprises 60-62% crude protein, 7-9% fiber, 8-12% WSDS, 5-7% fat and a small amount of the residual yeast. For removal of WSDS from biomass in an amount of 70-80% of their content in the biomass, an aqueous-ethanol solution, obtained in the process of the removal of the WSDS from biomass, containing mainly yeast is used (see below- the biomass containing mainly yeasts recovery from beer). The biomass is mixed with aqueous-ethanol solution in a ratio of 1.0: 2.4-2.6 and the resulting suspension is separated from the biomass by a decanter. The resulting biomass containing 20-23% of the dry matter is sent to drying, and the resulting aqueous-ethanol solution containing 0.8-1.0% WSDS (by weight of the solution) is passed to distillation.

### The biomass containing mainly yeasts recovery from beer and the removal of the WSDS from the biomass.

After the biomass containing mainly grain protein had been recovered from beer, the biomass containing mainly yeast was recovered from the beer by separation using a sedicanter. The recovered biomass contains 14-16% of dry matter. The dry matter contains 50-55% of yeasts, 20-25% of grain protein and 14-16% of WSDS. To remove the WSDS from the biomass in an amount of 80-90% of their content in the biomass, an aqueous-ethanol solution mixed in a ratio of 1.0:2.9-3.1 water-ethanol condensate, obtained by drying the biomasses and water condensate, obtained by evaporation of the stillage is used. The biomass is mixed with the aqueous-ethanol solution in a ratio of 1.0:9.5-10.5 and from the resulting suspension, the biomass is recovered by a sedicanter. The recovered biomass containing 15-17% of dry matter is passed to drying, and an aqueous-ethanol solution containing 0.2-0.4% (by weight of the solution) of WSDS is used for the removal of the WSDS from the biomass containing mainly grain protein.

### Drying the biomass containing mainly grain protein.

The resulting biomass containing 20-23% of dry matter is dried in a fluid bed dryer. As a heat carrier, at the inlet to the dryer, carbon dioxide heated to a temperature 110-160°C is used. At the exit of the dryer the temperature of the mixture of CO2 and water-ethanol vapor is maintained in the range of 75 - 100°C. After dryer the feed product is obtained in the form of granules, with a moisture content of 5-8%. Removal of WSDS from biomass in an amount of 70-80% of their content in biomass results in the reduced content of WSDS in the feed product, consequently increases the content of crude of protein in it from 60-62% (before removal of WSDS) to 65-68% (after removal of WSDS) by the weight of the product. In the process of drying the biomass, water-ethanol condensate is formed, part of which is taken from the dryer and used to remove the WSDS from the biomasses containing mainly yeast and grain protein. The other part of the condensate is used for CO2 regeneration.

### Drying biomass containing mainly yeast.

Yeast biomass containing 15-17% of dry matter is fed to a fluid bed dryer. When drying the biomass as a heat carrier at the inlet into the dryer carbon dioxide heated to a temperature 110-160°C is used. At the exit of the dryer the temperature of the mixture of CO2 and water ethanol vapor is maintained in the range 75 - 100°C. After dryer the feed product is obtained in the form of granules, with a moisture content of 5-8%. Removal of WSDS from biomass in an amount of 80-90% of their content in biomass results in the reduced content of WSDS in the feed product, consequently increases the content of crude of protein in it from 57-59% (before removal of WSDS) to 68-70% (after removal of WSDS). In addition to this the quality of protein in the feed product has significantly improved due to the removal of reducing carbohydrates that can react with amino acids of proteins in the process of drying biomass (Maillard reaction). In the process of drying the biomass, water ethanol condensate is formed, part of which is taken from the dryer and used to remove the WSDS from the biomasses containing mainly yeast and grain protein. The other part of the condensate is used for CO2 regeneration.

### Distillation of the clarified beer, and an aqueous-ethanol solution containing WSDS

Distillation of the clarified beer and the aqueous-ethanol solution containing WSDS is carried out separately. When distilling clarified beer the stillage with the concentration of the dry matter in the range of 2.7-3.0% and the distillate are obtained. When distilling the aqueous-ethanol solution the aqueous solution containing 1.1-1.3% of WSDS and ethanol condensate are obtained. The stillage is passed to evaporation, while the aqueous solution containing WSDS is used for enzymatic hydrolysis of the FGM. The distillate and ethanol condensate are combined and passed to rectification. When distillate is rectified in a mixture with ethanol condensate, ethanol and bottoms are obtained. Bottoms is used in enzymatic hydrolysis of FGM.

### Utilization of stillage and brans.

The stillage obtained by the distillation of the beer and containing 2.7-3.0% DM is evaporated by a vacuum evaporator to a dry matter content of 55-65%. The evaporated stillage is then mixed with brans in a ratio of 1.0:1.5-1.7 and the resulting mixture with a dry matter content of 75-80% is fed into a drum dryer. During the drying process the feed product is obtained with a moisture content of 8-11%, with a concentration of crude protein of 16-18%. Secondary water condensate obtained in the process of stillage evaporation is sent to the water treatment facilities.

Implementation example 2: by reducing WSDS, increasing the content of crude protein and its quality in feed product containing grain protein and yeast, by removing the WSDS from the biomasses recovered from the beer.

The sequence of all steps and their description in this implementation example before the step "The biomass containing mainly grain protein and the biomass containing mainly yeasts recovery from beer; the removal of the WSDS from the recovered biomasses" and after the step "Drying of the mixture of biomasses" are identical as in the implementation example 1. Additional operations and their description are given below.

### The biomass containing mainly grain protein and the biomass containing mainly yeasts recovery from beer; the removal of the WSDS from the recovered biomasses

The biomass containing mainly grain protein recovery from beer with followed removal of WSDS form the biomass in an amount of 70-80% of their content in the biomass is performed identically as described in the implementation example 1. The biomass containing mainly yeast recovery from beer with followed removal of WSDS from the biomass in an amount of 80-90% of their content in the biomass is performed identically as described in the implementation example 1. The recovered biomasses are passed to the mixer. To preserve the obtained mixture the ethanol is added in the amount of 11-13% by volume. The resulting biomass containing 16-18% of dry matter is fed to the buffer a tank, from which the biomass is sent to drying.

### Drying of the mixture of biomasses.

The resulting biomass mixture containing 16-18% of dry matter is dried in a fluid bed dryer. As a heat carrier at the inlet into the dryer carbon dioxide heated to a temperature 110-160°C is used. At the exit of the dryer the temperature of the mixture of CO2 and water ethanol vapor is maintained in the range 75 - 100°C. After dryer the feed product is obtained in the form of granules, with a moisture content of 5-8%. Removal of WSDS from biomass in an amount of 72-77% of their content in biomass results in the reduced content of WSDS in the feed product, consequently increases the content of crude of protein in it from 60-62% (before removal of WSDS) to 66-68% (after removal of WSDS) by the weight of the product. In addition to this the quality of protein in the feed product has significantly improved due to the removal of reducing carbohydrates that can react with amino acids of proteins in the process of drying biomass (Maillard reaction). In the process of drying the biomass, water-ethanol condensate is formed, part of which is taken from the dryer and used to remove the WSDS from the biomasses containing mainly yeast and grain protein. The other part of the condensate is used for CO2 regeneration.

Implementation example 3 : by obtaining the feed product containing mainly grain protein and the feed product containing components of degraded yeast, with a low content WSDS, with a high content of crude protein and its quality .

The sequence of all steps and their description in this implementation example before the step "The biomass containing mainly grain protein recovery from beer; the removal of the WSDS from the recovered biomass, and biomass drying" and after the step "Drying of the suspension of the degraded yeasts" are identical as in the implementation example 1. Additional operations and their description are given below.

### The biomass containing mainly grain protein recovery from beer; the removal of the WSDS from the recovered biomass, and biomass drying

The biomass containing mainly grain protein recovery from beer followed by the removal of WSDS from the biomass in an amount of 70-80% of their content in the biomass is performed identically as described in the implementation example 1.

### The biomass containing mainly yeast recovery from beer; the removal of the WSDS from the biomass with subsequent yeast degradation in biomass

The biomass containing mainly yeasts recovery from beer with subsequent removal of WSDS from the biomass in an amount of 80-90% of their content in the biomass is performed identically as described in the implementation example 1. The resulting biomass is diluted with water to its content of 8.5-9.5% DM and the nucleic acid reduction is then performed for 60-70 min at pH 6.9-7.5 and temperature 52-56°C. Then, the alkaline protease is applied to suspension in the amount of 0.1-0.12% by weight of the suspension and the further yeast degradation has been continued for 4-6 hours at the pH 7.5-8.5. Thus, yeast cells in the range of about 80% are degraded to unbound beta-glucan and mannan. Main part of intracellular yeast protein, in addition to grain protein which accompanies yeasts, is hydrolyzed mainly to amino acids, peptides and polypeptides. DM of the obtained suspension comprises crude protein in the range of 68-70%, beta-glucan 5-6%, mannan 3.5-4.5%, nucleotides 2.0-3.0%, as well as fat and minerals. The suspension of the degraded yeasts is fed to the dryer.

### Drying the suspension of the degraded yeasts

The suspension of the degraded yeast comprising 8.5-9.5% DM is passed to a spray dryer. When dry the suspension, a heat carrier at the inlet to the dryer, carbon dioxide heated to a temperature 150-250°C is used. At the exit from the dryer the temperature of the mixture of CO2 and water-ethanol vapor is maintained in the range of 75 - 100°C. After dryer the feed product is obtained in the form of dust-like particles, with a moisture content of 5-8%. The resultant feed product has the following positive characteristics: low content of WSDS and high content of crude protein ranging in the range of 68-70 %; polypeptides and peptides are of high quality due to removing reducing carbohydrates in the range of 80-90% of their content in biomass (preferably 80-85%), which can react with amino acids of these compounds (Maillard reaction); amino acids, peptides, polypeptides, unbound beta-glucan (4.5-5.5% by weight of the product) and mannan (3-4% by weight of the product), as well as nucleotides (2-3% by weight of the product) positively affect the quality of the feed product. During the drying of the suspension the water- ethanol condensate forms. A part of the formed condensate with the ethanol concentration of 1-1.2% by volume is taken from a drying unit and is used for enzymatic hydrolysis of FGM. Another part of the condensate is used for CO2 regeneration.

Implementation example 4: when obtaining the feed product containing mainly grain protein and components of degraded yeast, with a low content of WSDS, with a high content of crude protein and its quality.

The sequence of all steps and their description in this implementation example before the step "The biomass containing mainly grain protein recovery from beer and the removal of the WSDS from the biomass" and after the step "Drying of biomass containing mainly grain protein mixed with a suspension of degraded yeasts" are identical as in the implementation example 1. Additional operations and their description are given below.

### The biomass containing mainly grain protein recovery from beer and the removal of the WSDS from the biomass

The biomass containing mainly grain protein recovery from beer with subsequent removal of WSDS from the biomass in an amount of 70-80% of their content in the biomass is performed identically as described in the implementation example 1. The resulting biomass is fed to a mixer for mixing it with a suspension of degraded yeasts.

### The biomass containing mainly yeast recovery from beer; the removal of the WSDS from the biomass with subsequent yeast degradation in biomass

The biomass containing mainly yeasts recovery from beer; the removal of WSDS from the biomass in an amount of 80-90% of their content in the biomass with subsequent yeast degradation is performed identically as described in the implementation example 3. The resulting suspension is fed to a mixer for mixing it with the biomass containing mainly grain protein.

### Drying of biomass containing mainly grain protein mixed with a suspension of degraded yeasts

The suspension of the degraded yeasts is mixed with the biomass containing mainly grain protein in a mixer. To preserve the obtained mixture the ethanol is added in the amount of 11-13% by volume of biomass. The obtained biomass containing 14-16% DM is passed to a buffer vessel, from which the biomass is fed to a dryer. As a heat carrier, at the inlet to the dryer, carbon dioxide heated to a temperature 110-160°C is used. At the exit from the dryer the temperature of the mixture of CO2 and aqueous-ethanol vapor is maintained in the range of 75 - 100°C. After dryer the feed product is obtained in the form of granules, with a moisture content of 5-8%. The obtained feed product demonstrates following positive properties: low content of WSDS in the product and high crude protein content (66-68% by the weight of product); polypeptides and peptides are of high quality due to removing reducing carbohydrates in the range of 72-77 % of their content in biomass, which can react with amino acids of these compounds during drying biomass (Maillard reaction); amino acids, peptides, polypeptides, unbound beta-glucan (1.0-1.3 % of product weight) and mannan (0.6-0.8% of product weight), as well as nucleotides (0.4-0.6% of product weight) positively affect the quality of the feed product. During the drying of the mixture of biomasses the water- ethanol condensate forms. Part of the formed condensate is taken from a drying unit and is used in removing WSDS from biomasses containing mainly yeast and grain protein. Another part of the condensate is used for CO2 regeneration.

## Claims

1. A method for producing ethanol and feed products comprising the processing operations of grain grinding, recovering of brans and flour-grist mixture from the ground grain, enzymatic hydrolysis of flour-grist mixture to obtain mash, mash fermentation to obtain beer, the biomass containing mainly grain protein recovery from beer, **characterized in that** it additionally comprises the homogenization of biomass and where from the biomass, containing mainly grain protein and is recovered from beer, the water soluble dry solids (WSDS) are additionally removed in the amount of 65-85%, preferably 70-80%, of the total weight of water soluble dry solids contained in the biomass recovered from the beer.

2. The method according to claim 1 **characterized in that** the biomass recovered from beer, containing mainly grain protein, after removal of WSDS is dried, obtaining a feed product with a moisture content in the range of 3-10%, preferably 5-8%, comprising crude protein 58-72% by weight in the product, preferably 60-68%.

3. The method according to claim 1 or 2 **characterized in that** the additional recovery from beer the biomass containing mainly yeasts is performed, followed by mixing this biomass with the biomass containing mainly grain protein, in which WSDS have been removed in the amount of 65-85% of their content in the biomass, preferably 70-80%, and drying of the mixture of these biomasses, obtaining a feed product with a moisture content in the range of 3-10%, preferably 5-8%, comprising crude protein 58-72% by weight in the product, preferably 60-68%.

4. The method according to claim 1 or 3 **characterized in that** WSDS in an amount of 75-95% of their content in the biomass, preferably 80-90%, are removed from the biomass, containing mainly yeast and is recovered from the beer, followed by mixing this biomass with a biomass containing mainly grain protein in which the amount of WSDS have been removed in an amount of 65-85% of their content in the biomass, preferably 70-80%, and passing the mixture of these biomasses into drying, obtaining a feed product containing of 58-72%, preferably 60-68% by weight of crude protein in the product.

5. The method according to claim 1, 3 or 4 **characterized in that** in the biomass containing mainly yeasts and is recovered from beer, after WSDS removal in an amount of 75-95% of their content in the biomass, preferably 80-90%, further degradation of the yeast is performed to the biological active components and hydrolysis of the grain protein, accompanying yeast, to amino acids, peptides and polypeptides, followed by mixing the resulting suspension with biomass containing mainly grain protein in which the amount of WSDS have been removed in an amount of 65-85% of their content in the biomass, preferably 70-80%, and the mixture of these biomasses is dried, obtaining a feed product with a moisture content of 3-10%, preferably 5-8%, comprising crude protein in the product in the range of 58-72% by weight, preferably 60-68%.

6. The method according to any of claims 1-5 **characterized in that** to remove WSDS from a biomass containing mainly yeast, an aqueous-ethanol solution obtained by mixing the water condensate obtained by evaporating the stillage and the water-ethanol condensate obtained by drying the biomasses, and to remove WSDS from the biomass containing mainly grain protein, an aqueous-ethanol solution obtained by removing WSDS from the biomass containing mainly yeast is used.

7. The method according to any of claims 1-6 **characterized in that** to remove WSDS from a biomass containing mainly yeasts, an aqueous-ethanol solution obtained by mixing water and water-ethanol condensate obtained by drying the biomasses is used, and to remove WSDS from a biomass containing mainly grain protein, an aqueous-ethanol solution obtained by removing WSDS from a biomass containing mainly yeast is used.

8. The method according to any of claims 1-7 **characterized in that** the aqueous-ethanol solution obtained by removing WSDS from the biomasses is distilled separately from the clarified beer, and the resulting aqueous solution containing mainly WSDS is used in the enzymatic hydrolysis of the flour-grist mixture.

9. The method according to any of claims 1-8 **characterized in that** the stillage is evaporated to a DM content of 50-85% and then the evaporated stillage is dried together with bran to obtain a feed product.
